# EUROPÄISCHE PATENTSCHRIFT

(11) **EP 3 076 891 B1**
(45) Veröffentlichungstag und Bekanntmachung des Hinweises auf die Patenterteilung: **13.12.2017**
(21) Anmeldenummer: 14833338.8
(22) Anmeldetag: 10.11.2014
(51) Int. Cl.: A61B 34/00, A61B 34/30

(54) **ANTRIEBSANORDNUNG FÜR EIN ENDOSKOPISCHES SCHAFTINSTRUMENT**
DRIVE ARRANGEMENT FOR AN ENDOSCOPIC SHAFT-TYPE INSTRUMENT
DISPOSITIF D'ENTRAÎNEMENT POUR UN INSTRUMENT ENDOSCOPIQUE À TIGE

(30) Priorität: 06.12.2013 DE 102013225117
(43) Veröffentlichungstag der Anmeldung: 12.10.2016
(73) Patentinhaber: Richard Wolf GmbH, 75438 Knittlingen (DE)
(72) Erfinder: WEHRHEIM, Frank, 75015 Bretten (DE); SCHWEIGERT, Alexander, 75239 Eisingen (DE); PRESTEL, Stephan, 76287 Rheinstetten (DE); MÜNNIG, Sören, 75045 Walzbachtal (DE); LAMBERTZ, Matthias, 75015 Bretten (DE); KÖRNER, Eberhard, 75438 Knittlingen (DE)
(74) Vertreter: Patentanwälte Vollmann & Hemmer
(86) Internationale Anmeldenummer: PCT/DE2014/200625
(87) Internationale Veröffentlichungsnummer: WO 2015/081947

(56) Entgegenhaltungen:
- EP-A1- 0 054 763
- WO-A1-2013/159932
- WO-A2-2007/146987

## Beschreibung

Die Erfindung betrifft eine Antriebsanordnung für ein endoskopisches Schaftinstrument mit den im Anspruch 1 angegebenen Merkmalen.
Seit geraumer Zeit werden im Bereich der minimalinvasiven Chirurgie Operationsroboter eingesetzt. Diese Operationsroboter sind zumindest mit einem, in der Regel aber mit mehreren Roboterarmen ausgestattet, an deren distalen Enden jeweils ein von dem Operateur von einer Konsole aus gesteuertes endoskopisches Schaftinstrument angeordnet ist. Als endoskopische Schaftinstrumente werden im Folgenden derartige medizinische Instrumente verstanden, welche zum Beobachten, Manipulieren oder einer Kombination aus derartigen Funktionen zur Anwendung an oder im Körper eines Lebewesens verwendet werden. Dokument WO2013159932 A1 offenbart ein Chirurgierobotersystem nach dem Oberbegriff des Anspruchs 1. Ein weiterer Operationsroboter ist beispielsweise aus US 2009/0234371 A1 bekannt.
Die bei diesem Operationsroboter verwendeten Schaftinstrumente weisen an ihrem jeweiligen distalen Schaftende einen Instrumentenkopf mit einem daran angeordneten Werkzeug auf. In Verbindung mit den Schaftinstrumenten werden Instrumentenköpfe eingesetzt, die relativ zu dem Schaft abwinkelbar sind, wobei auch das Werkzeug bzw. ein an dem Instrumentenkopf vorgesehener Werkzeugträger mit dem Werkzeug gegenüber dem Instrumentenkopf abwinkelbar ist. Zur Steuerung der Abwinkelung des Instrumentenkopfes und zur Steuerung des Werkzeugs bzw. zur Aktuierung von Instrumentenkopf und Werkzeug werden Seilzüge verwendet, die durch den Schaft in ein an dem proximalen Schaftende angeordnetes Instrumentengehäuse geführt sind. Dort sind die Seilzüge an Betätigungsrollen befestigt, die jeweils mittels eines Betätigungsmotors gesteuert drehbar sind. Die Betätigungsrollen sind in dem Instrumentengehäuse in einer gemeinsamen Ebene normal zu ihrer Drehachse nebeneinander angeordnet.

Typischerweise wirkt sich die durch die Anzahl der Bewegungsfreiheitsgrade des Instrumentenkopfes bestimmte Anzahl der in dem Instrumentengehäuse angeordneten Betätigungsrollen in erheblicher Weise auf die Größe des Instrumentengehäuses aus. So ist das Instrumentengehäuse bei einem Schaftinstrument, in dessen Instrumentengehäuse lediglich vier Betätigungsrollen angeordnet sind, schon relativ groß. Diese Größe des Instrumentengehäuses erweist sich dann als nachteilig, wenn mehrere dieser Schaftinstrumente auf engem Raum gemeinsam eingesetzt werden müssen, wie es beispielsweise bei Single-Port-Eingriffen der Fall ist, bei denen die Schaftinstrumente dem Operationsgebiet gleichzeitig über eine gemeinsame Körperöffnung zugeführt werden. Des Weiteren erweist sich ein großes Instrumentengehäuse dann als hinderlich, wenn es erforderlich ist, den Schaft dieser Instrumente in einem möglichst flachen Winkel zur Körperoberfläche eines Patienten in dessen Körper einzuführen.

Vor diesem Hintergrund besteht die Aufgabe der Erfindung darin, eine Antriebsanordnung für ein endoskopisches Schaftinstrument der oben genannten Art zu schaffen, die ein hinsichtlich Formgebung und/oder Größe verbessertes Instrumentengehäuse ermöglicht und das Einsatzspektrum des Schaftinstruments vergrößert.

Diese Aufgabe wird durch eine Antriebsanordnung mit den in Anspruch 1 angegebenen Merkmalen gelöst. Vorteilhafte Weiterbildungen dieser Antriebsanordnung ergeben sich aus den Unteransprüchen, der nachfolgenden Beschreibung sowie den Zeichnungen. Gemäß der Erfindung können hierbei die Unteransprüche jeweils für sich, aber auch in sinnvoller Kombination miteinander die Antriebsanordnung nach Anspruch 1 weiter ausgestalten.

Die erfindungsgemäße Antriebsanordnung für ein endoskopisches Schaftinstrument, bei dem es sich vorzugsweise um ein medizinisches Instrument handelt, das zum Einsatz in Verbindung mit einem medizinischen Operationsroboter vorgesehen ist, weist ein Instrumentengehäuse am proximalen Schaftende auf. In dem Instrumentengehäuse ist zumindest eine, bevorzugt vier, Gehäusewelle(n) gelagert. Sofern es die Anzahl der zu steuernden Bewegungsfreiheitsgrade für den distalen Instrumentenkopf erfordert, können auch mehr als vier Gehäusewellen angeordnet werden. Diese sind mit Zugmitteln abtriebsverbunden, die zur Steuerung eines Instrumentenkopfes am distalen Schaftende vorgesehen sind. Als Zugmittel dienen bevorzugt Seilzüge, die durch den Instrumentenschaft geführt sind oder direkt im Instrumentengehäuse wirksam werden. Bei den Gehäusewellen handelt es sich um die Wellen, welche die an sich bekannten, in dem Instrumentengehäuse angeordneten Betätigungsrollen für die Zugmittel tragen. Weiter weist die erfindungsgemäße Antriebsanordnung eine Antriebseinheit mit zumindest einer, bevorzugt vier, parallel zueinander ausgerichteten Antriebswellen auf. Sofern es die Anzahl der zu steuernden Bewegungsfreiheitsgrade für den distalen Instrumentenkopf erfordert, können auch mehr als vier Antriebswellen angeordnet werden. Mit jeder dieser Antriebswellen ist jeweils eine der Gehäusewellen antriebsverbindbar.

Die Grundidee der Erfindung besteht darin, die Gehäusewellen zu den mit ihnen antriebsverbindbaren Antriebswellen schräg auszurichten. Hierbei ist die Richtung, in der die Gehäusewellen zu den Antriebswellen schräg ausgerichtet sind, zunächst grundsätzlich beliebig. Bevorzugt ist vorgesehen, alle Gehäusewellen schräg zu stellen. Diese Schrägstellung der Gehäusewellen erlaubt eine große Gestaltungsfreiheit hinsichtlich der Formgebung des Instrumentengehäuses und bei entsprechender Ausrichtung der Gehäusewellen die Schaffung von vergleichsweise kleinbauenden Instrumentengehäusen. Darüber hinaus kann eine Gesamteinheit von Instrumentengehäuse und Antriebseinheit bei geeigneter Schrägstellung der Gehäusewellen so gestaltet werden, dass das Schaftinstrument bei solchen Eingriffen zum Einsatz kommen kann, bei denen der Schaft dieser Instrumente in einem möglichst flachen Winkel zur Körperoberfläche eines Patienten in dessen Körper eingeführt werden muss.

Bei einer bevorzugten Ausgestaltung mit vier Gehäusewellen sind diese vorteilhafterweise paarweise in Richtung der Längsachse des Instrumentenschaftes hintereinander an zwei voneinander abgewandten Seiten des Instrumentenschaftes angeordnet. Hierbei sind die Gehäusewellen bevorzugt derart angeordnet, dass die Enden der Gehäusewellen, an denen diese mit den Antriebswellen antriebsverbindbar sind, die Eckpunkte eines Rechtecks ergeben, wobei der Schaft des Schaftinstrumentes in einer zum Rechteck parallelen Ebene angeordnet ist.

Besonders vorteilhaft ist eine Formgebung des Instrumentengehäuses, bei dem sich dieses in einer Ebene normal zur Längsausdehnung des Instrumentenschaftes zu einer Spitze verjüngt. Hierbei ist der Instrumentenschaft zweckmäßigerweise in unmittelbarer Nähe der an dem Instrumentengehäuse ausgebildeten Spitze angeordnet. Diese Ausgestaltung ermöglicht es, mehrere Schaftinstrumente auf engstem Raum, zum Beispiel bei einem Single-Port-Eingriff, gleichzeitig zu benutzen. So können beispielsweise dann, wenn sich das Instrumentengehäuse in einem Winkel von 120° zuspitzt, bereits drei Schaftinstrumente in unmittelbarer Nähe zueinander eingesetzt werden. Durch Verkleinerung des Winkels besteht typischerweise die Möglichkeit, gegebenenfalls noch mehr Schaftinstrumente auf engstem Raum nebeneinander einzusetzen. Insbesondere im Hinblick auf eine raumsparende Anordnung der Gehäusewellen und der damit verbundenen Betätigungsrollen in dem Instrumentengehäuse ist eine Ausgestaltung von Vorteil, bei der die Gehäusewellen in von dem Instrumentenschaft weg weisender Richtung schräggestellt sind. Dies gilt insbesondere dann, wenn, wie es bevorzugt vorgesehen ist, die Gehäusewellen paarweise in Richtung der Längsachse des Instrumentenschaftes hintereinander an zwei voneinander abgewandten Seiten des Instrumentenschaftes angeordnet sind und die Enden der Gehäusewellen, an denen diese mit den Antriebswellen antriebsverbindbar sind, die Eckpunkte eines Rechtecks bilden, wobei der Schaft des Schaftinstrumentes in einer zum Rechteck parallelen Ebene angeordnet ist. In diesem Fall sind die Gehäusewellen vorzugsweise derart schräggestellt, dass sie in Richtung normal zu der Längsausdehnung des Instrumentenschaftes schräg nach außerhalb der von den Enden der Gehäusewellen aufgespannten Fläche verlaufen. Hierdurch lässt sich besonders günstig eine Ausgestaltung verwirklichen, bei der sich das Instrumentengehäuse in einer Ebene normal zur Längsausdehnung des Instrumentenschaftes zu einer Spitze verjüngt. Des Weiteren wird hierbei trotz einer an sich kompakten Ausgestaltung genug freier Raum bereit gestellt, in dem vorteilhafterweise eine parallel zur Längsausdehnung des Instrumentenschaftes ausgerichtete Linearführung angeordnet sein kann, an deren Schiene distal ein Trokar an das Schaftinstrument angekoppelt werden kann.

Alternativ zu der beschriebenen Schrägstellung der Gehäusewellen können diese gemäß einer weiteren vorteilhaften Ausgestaltung der erfindungsgemäßen Antriebsanordnung auch jeweils in einer parallel zur Längsausdehnung des Instrumentenschaftes ausgerichteten Ebene, in der die Antriebswellen liegen, abgeschrägt ausgerichtet sein. Diese Schrägstellung der Gehäusewellen ist zum Beispiel dann von Nutzen, wenn das Schaftinstrument so an einem Roboterarm angeordnet werden soll, dass der von dem Schaft des Instrumentes und einer Mittelachse des Roboterarms eingeschlossene Winkel von 90° abweicht. Bei entsprechender Winkelauslegung erlaubt diese Ausgestaltung eine Anordnung des Schaftinstruments an einem Roboterarm, bei der der Instrumentenschaft in einem vergleichsweise flachen Winkel zur Körperoberfläche eines Patienten in dessen Körper eingeführt werden kann.

Grundsätzlich ist die Art der Antriebsverbindung der Gehäusewellen mit den Antriebswellen beliebig. Eine besonders kompakte Gesamteinheit von Instrumentengehäuse und Antriebseinheit lässt sich aber dann verwirklichen, wenn, wie es bevorzugt vorgesehen ist, die Gehäusewellen jeweils über zumindest ein Kardangelenk oder andere winkelausgleichende Gelenke, wie z. B. Podegelenke, mit den Antriebswellen bewegungsgekoppelt sind. Darüber hinaus ist mit der Verwendung dieser Gelenkarten ein vergleichsweise hoher Wirkungsgrad bei der Bewegungsübertragung von der Antriebswelle auf die Gehäusewelle verbunden.

Typischerweise ist es vorgesehen, dass die Antriebsverbindung der Gehäusewellen mit den Antriebswellen in einfacher und schneller Weise hergestellt und wieder getrennt werden kann. Im Folgenden wird der Begriff des Kardangelenkes stellvertretend für winkelausgleichende Gelenkwellen verwendet. In diesem Zusammenhang erweist es sich als zweckmäßig, wenn das Kardangelenk einen mit der Gehäusewelle verbundenen ersten Teil und einen mit der Antriebswelle verbundenen zweiten Teil aufweist, wobei der erste und der zweite Teil des Kardangelenks mittels einer Steckverbindung miteinander verbindbar sind. Um die Steckverbindung des ersten Teils des Kardangelenks mit dessen zweiten Teil zu ermöglichen, weist das ansonsten geschlossen ausgebildete Instrumentengehäuse Öffnungen auf, über welche die mit den Gehäusewellen verbundenen Teile des Kardangelenks von außen zugänglich sind. Hierbei ist vorzugsweise jeder Gehäusewelle eine an dem Instrumentengehäuse ausgebildete Öffnung zugeordnet.

Vorteilhafterweise weist auch die Antriebseinheit ein im Wesentlichen geschlossenes Antriebsgehäuse auf. In dem Antriebsgehäuse sind alle Antriebswellen und die diesen zugeordneten Antriebsmotoren angeordnet. Zur kompakten Gestaltung des Antriebsgehäuses sind die Antriebsmotoren bevorzugt zueinander parallel ausgerichtet. Jede Antriebswelle ist mit dem jeweils zugehörigen Antriebsmotor bevorzugt axial fluchtend ausgerichtet. An dem Antriebsgehäuse sind zweckmäßigerweise Öffnungen ausgebildet, über welche die mit den Antriebswellen verbundenen Teile des Kardangelenks zugänglich sind. Bevorzugt ist das Antriebsgehäuse mit dem Instrumentengehäuse mittels einer Steckverbindung verbindbar. Demnach sind an den miteinander zur Anlage kommenden Fügeflächen von Instrumentengehäuse und Antriebsgehäuse, an denen auch die Öffnungen für die jeweiligen Teile des Kardangelenks ausgebildet sind, Steckelemente vorgesehen, die beim Zusammenfügen von Instrumentengehäuse und Antriebsgehäuse miteinander in Eingriff treten. Vorteilhafterweise ist das Antriebsgehäuse derart dimensioniert, dass die antriebsgehäuseseitige Fügefläche mit der an dem Instrumentengehäuse ausgebildeten Fügefläche korrespondiert, sodass die beiden Fügeflächen eine formschlüssige Verbindung eingehen.

Um sicherzustellen, dass beim Zusammenfügen von Instrumentengehäuse und Antriebsgehäuse auch eine Antriebsverbindung der in dem Instrumentengehäuse angeordneten Gehäusewellen mit den in dem Antriebsgehäuse angeordneten Antriebswellen stattfindet, sind in dem Instrumentengehäuse gemäß einer weiteren vorteilhaften Weiterbildung der Erfindung Federelemente vorgesehen, mit denen die in dem Instrumentengehäuse gelagerten Gehäusewellen bei von dem Antriebsgehäuse getrenntem Instrumentengehäuse in einer festgelegten Drehposition festlegbar sind. Ziel dieser Maßnahme ist es, den an den Gehäusewellen angeordneten Teil jedes Kardangelenks so zu positionieren, dass er mit dem an den Antriebswellen angeordneten Teil des Kardangelenks sofort durch Zusammenstecken verbindbar ist. Typischerweise ist hierbei auch sicherzustellen, dass sich die mit den Antriebswellen verbundenen Teile der Kardangelenke in einer entsprechenden Position befinden. Hier ist es aber möglich, diese Teile der Kardangelenke durch entsprechende Ansteuerung der Antriebsmotoren in eine solche Stellung zu verfahren.

Zur Festlegung der Gehäusewellen bei von dem Antriebsgehäuse getrenntem Instrumentengehäuse ist in dem Instrumentengehäuse vorteilhafterweise jeder der dort angeordneten Gehäusewellen ein Blattfederelement zugeordnet, das jeweils in einer Verriegelungsstellung in eine im Bereich der Außenseite des an der jeweiligen Gehäusewelle angeordneten ersten Teils des Kardangelenks ausgebildete Ausnehmung eingreift. Die Blattfederelemente sind zweckmäßigerweise so angeordnet, dass sie bei mit dem Antriebsgehäuse verbundenem Instrumentengehäuse von dem Antriebsgehäuse vorgespannt werden und sich somit nicht im Eingriff befinden. Nach dem Trennen des Antriebsgehäuses von dem Instrumentengehäuse können die Gehäusewellen manuell so verdreht werden, dass die Position der an den Teilen des Kardangelenks ausgebildeten Ausnehmung mit der Position des zugeordneten Blattfederelements übereinstimmt, sodass das Blattfederelement unter Federentspannung in die Ausnehmung eingreift. Alternativ hierzu ist es auch möglich die Gehäusewellen vor der Trennung des Antriebsgehäuses von dem Instrumentengehäuse durch eine entsprechende Ansteuerung der dann mit den Gehäusewellen bewegungsgekoppelten Antriebsmotoren in eine Stellung zu verfahren, in der die Position der an den Teilen des Kardangelenks ausgebildeten Ausnehmung mit der Position des zugeordneten Blattfederelements übereinstimmt.

Um die Verriegelung der Gehäusewellen, die durch das Eingreifen der Blattfederelemente in die an den ersten Teilen der Kardangelenke der Gehäusewellen ausgebildeten Ausnehmungen verursacht wird, zu lösen, sind an dem Antriebsgehäuse vorteilhafterweise in Fügerichtung von Instrumentengehäuse und Antriebsgehäuse vorstehende Vorsprünge ausgebildet, die beim Zusammenfügen von Instrumentengehäuse und Antriebsgehäuse die in dem Instrumentengehäuse vorgesehenen Blattfederelemente in eine Entriegelungsstellung drücken. Dementsprechend erfolgt die Entriegelung der Gehäusewellen allein durch das Zusammenfügen von Instrumentengehäuse und Antriebsgehäuse.

Neben der Möglichkeit, durch das Festlegen der Gehäusewellen zu gewährleisten, dass sich die an den Gehäusewellen angeordneten ersten Teile der Kardangelenke in einer Stellung befinden, in der sie mit den an den Antriebswellen angeordneten zweiten Teilen des Kardangelenks zusammengefügt werden können, kann dies ebenso vorteilhaft dadurch sichergestellt werden, dass die Antriebswellen im Antriebsgehäuse in axialer Richtung auf Federelementen gelagert sind und gegen Federkraft in Fügerichtung von Instrumentengehäuse und Antriebsgehäuse axial verschiebbar sind. Alternativ hierzu kann die federnde Anordnung umgekehrt werden, sodass die Gehäusewellen in dem Instrumentengehäuse in axialer Richtung auf Federelementen gelagert sind und gegen Federkraft in Fügerichtung von Instrumentengehäuse und Antriebsgehäuse axial verschiebbar sind. Werden bei diesen beiden Ausgestaltungen das Instrumentengehäuse und das Antriebsgehäuse zusammengefügt, kontaktieren die an den Antriebswellen angeordneten zweiten Teile der Kardangelenke die an den Gehäusewellen angeordneten ersten Teile der Kardangelenke, wobei die Antriebswellen von den an den Gehäusewellen angeordneten zweiten Teilen der Kardangelenke in Fügerichtung von Instrumenten- und Antriebsgehäuse weggeschoben werden oder alternativ die Gehäusewellen von den an den Antriebswellen angeordneten zweiten Teile der Kardangelenke in Fügerichtung von Instrument und Antriebseinheit weggeschoben werden, ohne dass die ersten und die zweiten Teile der Kardangelenke eine Steckverbindung bilden. Hierdurch werden die Federelemente, auf denen die Antriebswellen, oder alternativ die Gehäusewellen, gelagert sind, vorgespannt. Durch Anfahren der mit den Antriebswellen bewegungsgekoppelten Antriebsmotoren werden die Antriebswellen so weit relativ gegenüber den Gehäusewellen verdreht, bis sich die an den Antriebswellen angeordneten zweiten Teile der Kardangelenke in einer Stellung befinden, in der die an den Gehäusewellen angeordneten Teile der Kardanwelle mittels einer durch Entspannung der Federelemente verursachten Rückbewegung der Antriebswellen, oder alternativ der Gehäusewellen, in die an den Antriebswellen angeordneten zweiten Teile der Kardangelenke eingreifen können.

Zweckmäßigerweise ist sicherzustellen, dass das Instrumentengehäuse und das Antriebsgehäuse nur in einer definierten Position zueinander zusammengefügt werden können. Bevorzugt sind hierzu an den Fügeflächen von Instrumentengehäuse und Antriebsgehäuse Führungsmittel zur Lagefixierung des Antriebsgehäuses an dem Instrumentengehäuse vorgesehen. Konstruktiv einfach können an einem von Instrumenten- und Antriebsgehäuse zumindest zwei in Fügerichtung vorstehende Vorsprünge ausgebildet sein, die in an dem anderen Gehäuse ausgebildete Ausnehmungen eingreifen. Bei einer Ausgestaltung, bei der die Gehäusewellen mittels Blattfederelementen festlegbar sind und zum Lösen der Festlegung der Gehäusewellen an dem Antriebsgehäuse in Fügerichtung von Instrumentengehäuse und Antriebsgehäuse vorstehende Vorsprünge ausgebildet sind, können vorteilhaft auch diese Vorsprünge als Führungsmittel dienen.

Eine wiederholt lösbare und positionsstabile Verbindung von Antriebsgehäuse und Instrumentengehäuse wird bei der erfindungsgemäßen Antriebsanordnung vorzugsweise dadurch verwirklicht, dass an dem Instrumentengehäuse eine in Fügerichtung von Instrumentengehäuse und Antriebsgehäuse durch das Instrumentengehäuse verlaufende Passbohrung ausgebildet ist und an dem Antriebsgehäuse ausgehend von einer Fügefläche mit dem Instrumentengehäuse eine korrespondierende Passbohrung ausgebildet ist, wobei die an dem Antriebsgehäuse und dem Instrumentengehäuse ausgebildeten Passbohrungen zur Aufnahme eines Verschlussstiftes vorgesehen sind. Hierbei kann der Verschlussstift in seiner in die an dem Antriebsgehäuse ausgebildete Passbohrung eingreifenden Stellung an dem Antriebsgehäuse festgelegt sein und einen festen Bestandteil des Instrumentengehäuses bilden.

An dem Antriebsgehäuse ist zweckmäßigerweise eine die dort in Fügerichtung von Instrumentengehäuse und Antriebsgehäuse verlaufende Passbohrung kreuzende zweite Passbohrung ausgebildet, in welcher ein Verriegelungsstift verschiebbar geführt ist, der in einer Verriegelungsstellung in eine an dem Verschlussstift ausgebildete Ausnehmung eingreift. Hierdurch werden das Instrumentengehäuse und das Antriebsgehäuse fest miteinander verbunden, wobei ein an dem Ende des Verriegelungsstiftes ausgebildeter Betätigungskopf, welcher an der Außenseite des Antriebsgehäuses herausragt und somit leicht zugänglich ist, ein einfaches Lösen der Verbindung von Instrumentengehäuse und Antriebsgehäuse ermöglicht.

Nachfolgend ist die Erfindung anhand von in den Zeichnungen dargestellten Ausführungsbeispielen näher erläutert. In den Zeichnungen zeigt jeweils schematisch vereinfacht und in unterschiedlichen Maßstäben:
- Fig. 1: einen proximalen Endabschnitt eines endoskopischen Schaftinstruments mit verbundenem Antriebsgehäuse und Antriebsmotoren in einer perspektivischen Darstellung ohne Gehäuseteil des Instrumentes,
- Fig. 2: den proximalen Endabschnitt des endoskopischen Instru-ments nach Fig. 1 in einer perspektivischen Rückansicht ohne Darstellung jeglicher Arretierungsmechanismen zwischen Antriebseinheit und Instrument,
- Fig. 3: eine Antriebsanordnung für das endoskopische Schaftinstrument nach Fig. 1 in einer perspektivischen Ansicht ohne Gehäuseteil des Instrumentes sowie ohne das Antriebsgehäuse,
- Fig. 4: die Antriebsanordnung nach Fig. 3 in einer Draufsicht,
- Fig. 5: in einer geometrischen Prinzipskizze einen Antriebsstrang der Antriebsanordnung nach Fig. 3,

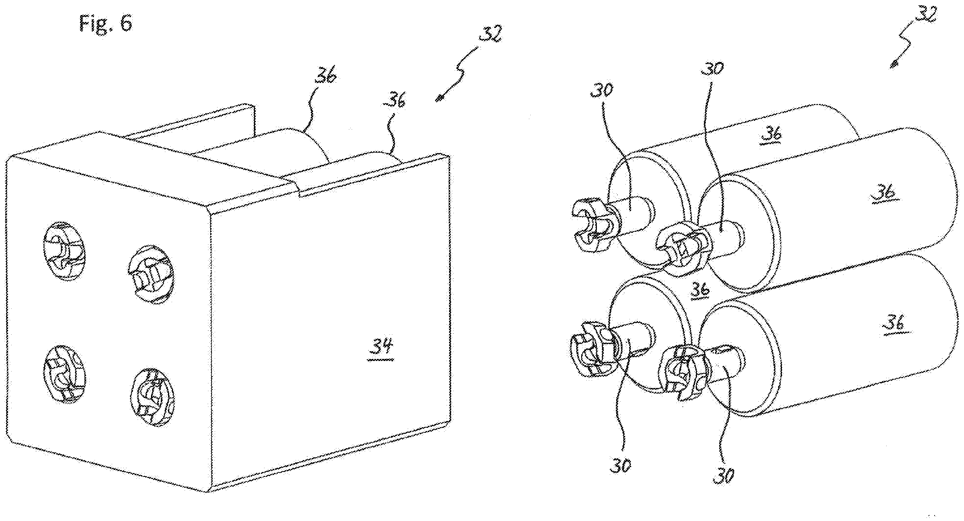

- Fig. 6: eine Anordnung von Antriebsmotoren der Antriebseinheit mit antriebsseitigen Kardanwellengelenkkomponenten für das endoskopische Schaftinstrument nach Fig. 1 in perspektivischer Darstellung,
- Fig. 7: ein Kardanwellengelenk der Antriebswellenanordnung nach Fig. 3 in perspektivischer Darstellung unter Vernachlässigung der Wellenlagerung,
- Fig. 8: ein Kardanwellengelenk der Antriebsanordnung nach Fig. 3 in perspektivischer Darstellung gemäß einer zweiten Ausführungsform,
- Fig. 9: eine Variante der Verbindungsstelle zwischen Instrument und Antriebseinheit der Antriebsanordnung nach Fig. 3 in einer perspektivischen Explosionsdarstellung in reduzierter Form,
- Fig. 10: eine Einzelheit A aus Fig. 9,
- Fig. 11: eine Einzelheit B aus Fig. 9,
- Fig. 12: eine Einzelheit C aus Fig. 9,
- Fig. 13: eine Einzelheit D aus Fig. 9,
- Fig. 14: eine in einem Antriebsgehäuse der Antriebseinheit nach Fig. 1 gelagerte Antriebswelle,
- Fig. 15: die Darstellung nach Fig. 14 in einer Schnittansicht,
- Fig. 16: eine Prinzipdarstellung der Antriebsanordnung nach Fig. 3 in einer perspektivischen Ansicht unter Vernachlässigung der Betätigungsrollen, des Instrumentenschaftes sowie des Instrumentenkopfes und detaillierter Gehäuseausgestaltungen,
- Fig. 17: die Darstellung nach Fig. 16 bei von einem Instrumentengehäuse getrennten Antriebsgehäuse,
- Fig. 18: die Darstellung nach Fig. 16 in einer Schnittansicht,
- Fig. 19: die Darstellung nach Fig. 17 in einer Schnittansicht,
- Fig. 20: in perspektivischer Darstellung eine Antriebsanordnung für ein an einem Roboterarm angeordnetes endoskopisches Schaftinstrument in einer zweiten Ausführungsform,
- Fig. 21: in perspektivischer Darstellung eine Antriebsanordnung in einer weiteren Ausführungsform,
- Fig. 22: in einer perspektivischen Darstellung ein an einem Roboterarm eines Operationsroboters angeordnetes endoskopisches Schaftinstrument nach einer weiteren Ausführungsform,
- Fig. 23: in einer perspektivischen Darstellung ein an einem zweiten Roboterarm eines Operationsroboters angeordnetes endoskopisches Schaftinstrument, und
- Fig. 24: in einer Prinzipdarstellung drei endoskopische Schaftinstrumente in Draufsicht mit einer Antriebsanordnung nach Fig. 3 bei einem Single-Port-Eingriff.

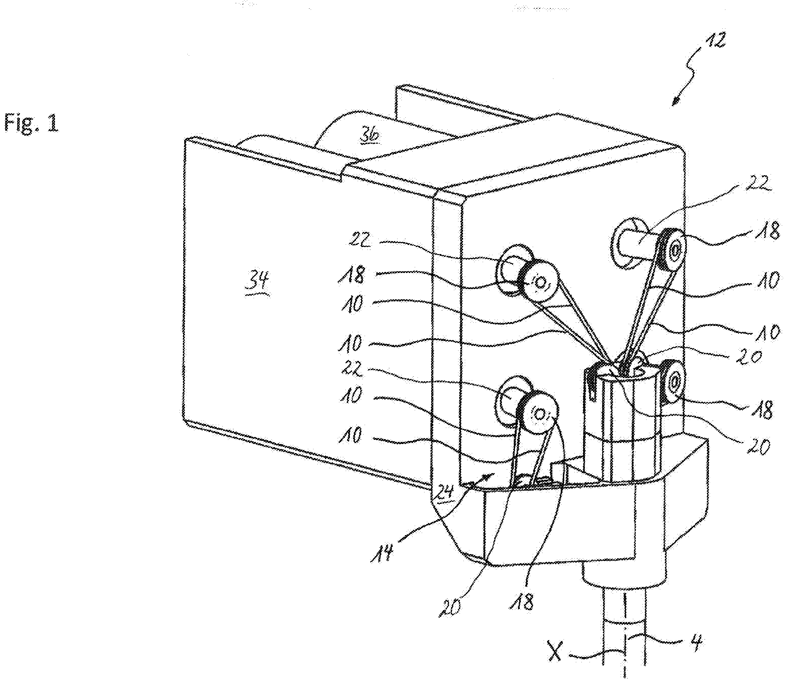

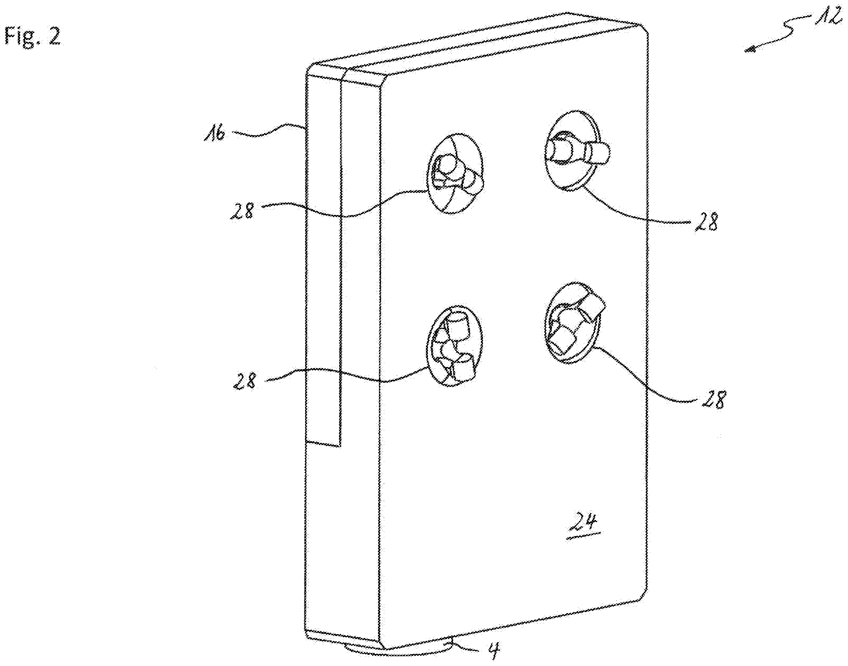

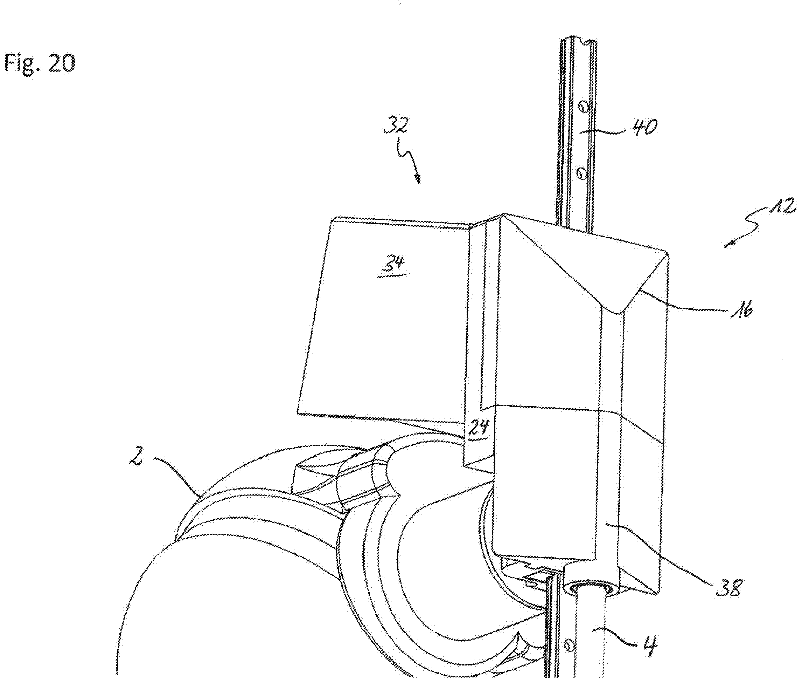

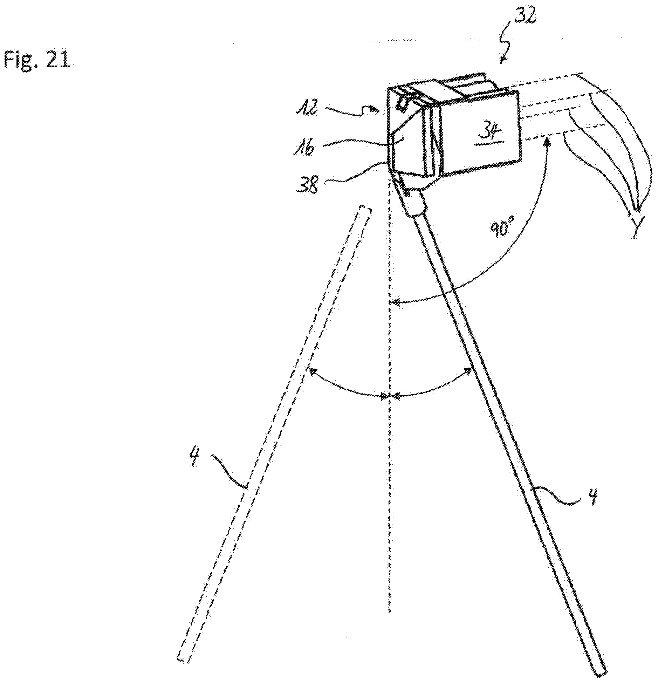

Das in den Fig. 1 und 2 dargestellte endoskopische Schaftinstrument ist zur Anordnung an einem Roboterarm 2 eines chirurgischen Operationsroboters vorgesehen (Fig. 20, 22 und 23). Es weist einen länglichen, starren Instrumentenschaft 4 auf. In den Fig. 1 und 2 ist der Instrumentenschaft 4 aus Gründen einer besseren Übersichtlichkeit nahezu vollständig weggelassen, er entspricht aber dem Instrumentenschaft 4 des in den Fig. 21 bis 23 dargestellten endoskopischen Schaftinstruments. Bei dem Instrumentenschaft 4 handelt es sich um einen Hohlschaft. Wie bei dem in den Fig. 21 bis 23 dargestellten Schaftinstrument ist auch an dem distalen Schaftende des in den Fig. 1 und 2 dargestellten Schaftinstruments ein Instrumentenkopf 6 mit einer Abwinkelungskinematik 7 und einem Werkzeug 8 angeordnet. Das Werkzeug 8 ist mittels der Abwinkelungskinematik 7 relativ zu dem Instrumentenschaft 4 abwinkelbar.

Zur Steuerung der Abwinkelung des Werkzeugs 8, bei dem es sich im vorliegenden Fall um ein Maulwerkzeug handelt, sind Zugmittel in Form von sechs Seilzügen 10 vorgesehen, die durch den Instrumentenschaft 4 in ein an dem proximalen Schaftende angeordnetes Instrumentengehäuse 12 geführt sind. Zur Steuerung der Rotation des Instrumentenschaftes 4 um seine Längsachse X sind zwei weitere Zugmittel in Form von Seilzügen 10 vorgesehen, die im Instrumentengehäuse 12 angeordnet sind.

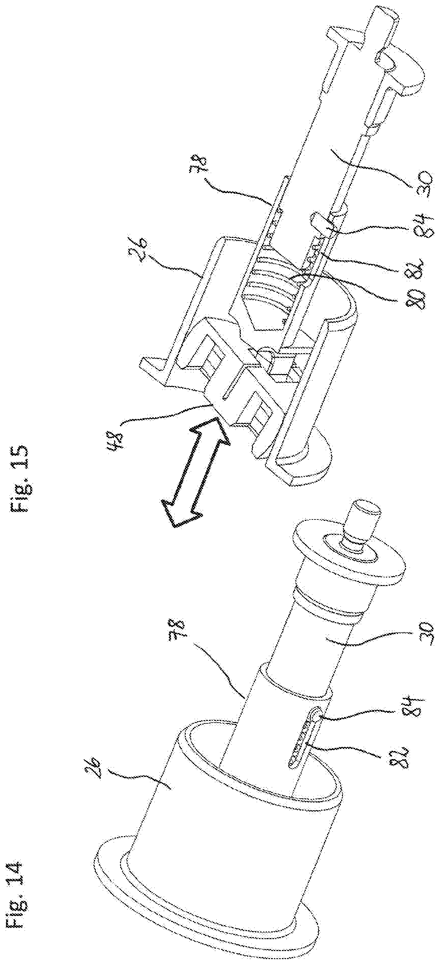

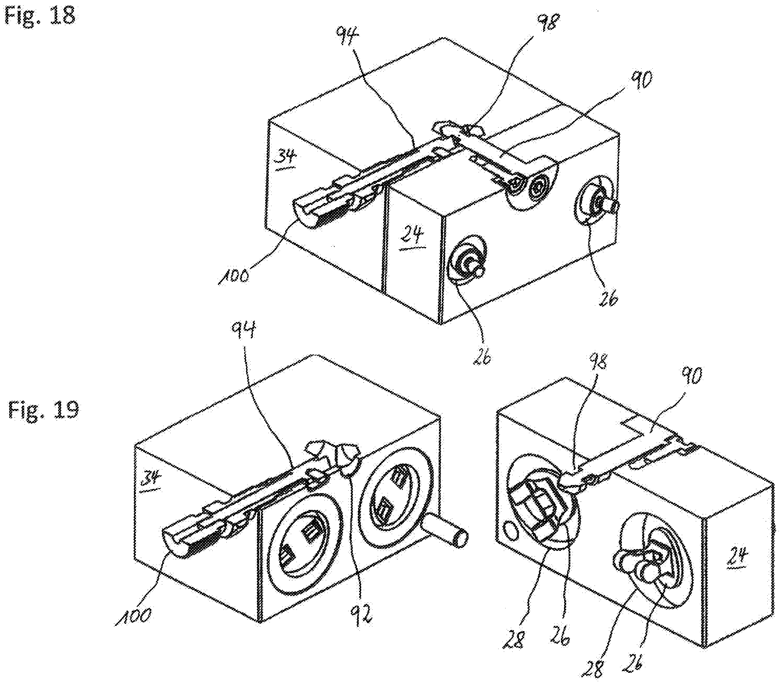

In einem in dem Instrumentengehäuse 12 ausgebildeten Freiraum 14, der von einem Gehäuseteil 16 des Instrumentengehäuses 12 überdeckt wird, sind die Seilzüge 10 paarweise an vier Betätigungsrollen 18 befestigt, wobei sie zuvor an in dem Freiraum 14 drehbar gelagerten Umlenkrollen 20 von dem Instrumentenschaft 4 in Richtung der Betätigungsrollen 18 umgelenkt sind. Die Betätigungsrollen 18 sind an den Enden von Gehäusewellen 22 befestigt, die durch einen parallel zur Längsachse X des Instrumentenschaftes 4 ausgerichteten plattenförmigen Abschnitt eines Grundkörpers 24 des Instrumentengehäuses 12 geführt sind und in diesem Abschnitt des Grundkörpers 24 in Lagerbuchsen 26 (Fig. 14 bis 19) drehbar gelagert sind. An dem Grundkörper 24 sind die Gehäusewellen 22 paarweise in Richtung der Längsachse X des Instrumentenschaftes 4 an zwei voneinander abgewandten Seiten des Instrumentenschaftes 4 angeordnet.

Die Gehäusewellen 22 sind vollständig durch den Grundkörper 24 hindurchgeführt, sodass die von den Betätigungsrollen 18 abgewandten Enden der Gehäusewellen 22 an der von dem Freiraum 14 abgewandten Rückseite des Grundkörpers 24 herausragen. Die an der Rückseite des Grundkörpers 24 herausragenden Enden der Gehäusewellen 22 sind gabelförmig ausgebildet und im Bereich von an dem Grundkörper 24 ausgebildeten zylindrischen Vertiefungen angeordnet, worauf nachfolgend noch ausführlicher eingegangen wird.

An den an der Rückseite des Grundkörpers 24 herausragenden Enden sind die Gehäusewellen 22 mit den Antriebswellen 30 einer Antriebseinheit 32 wiederholt lösbar antriebsverbindbar. In einem Antriebsgehäuse 34 der Antriebseinheit 32 ist jede der vier Antriebswellen 30 über in der Zeichnung nicht dargestellte Getriebemittel mit der Motorwelle eines Antriebsmotors 36 bewegungsgekoppelt.

Im antriebsverbundenen Zustand sind alle vier Antriebswellen 30 normal zur Längsachse X des Instrumentenschaftes 4 ausgerichtet. Die Gehäusewellen 22 sind bei allen in der Zeichnung dargestellten Ausführungsformen der Antriebsanordnung für ein endoskopisches Schaftinstrument schräg zu den Antriebswellen 30 ausgerichtet.

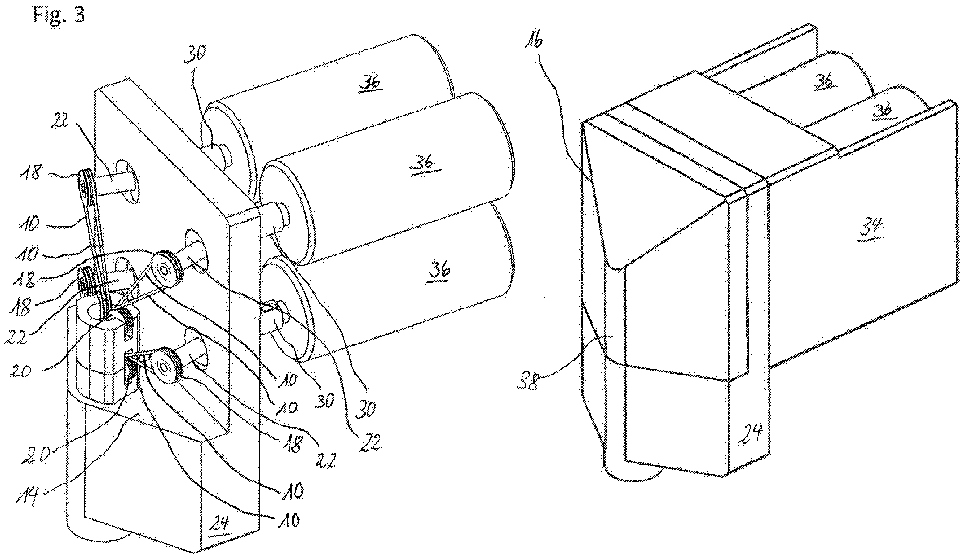

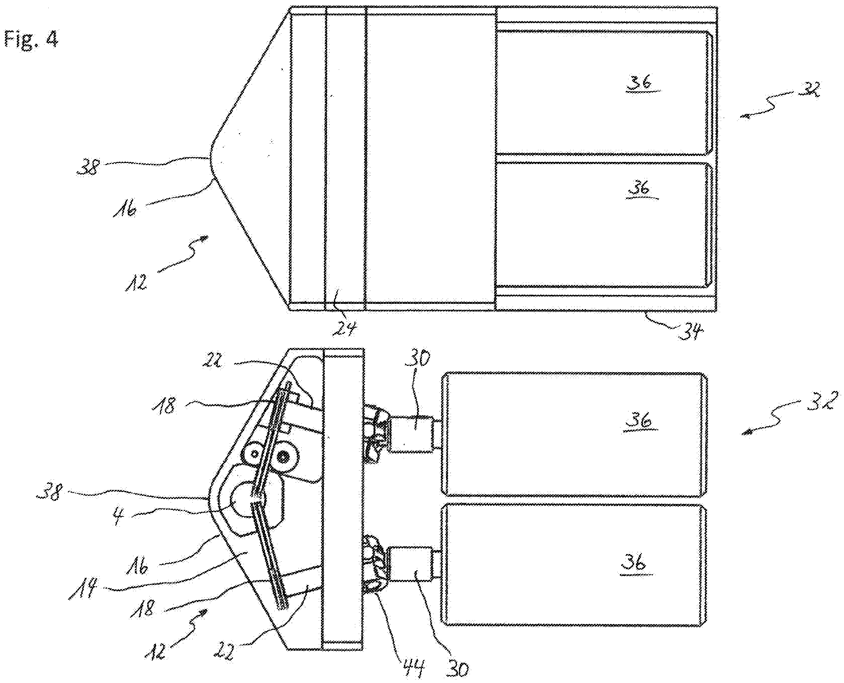

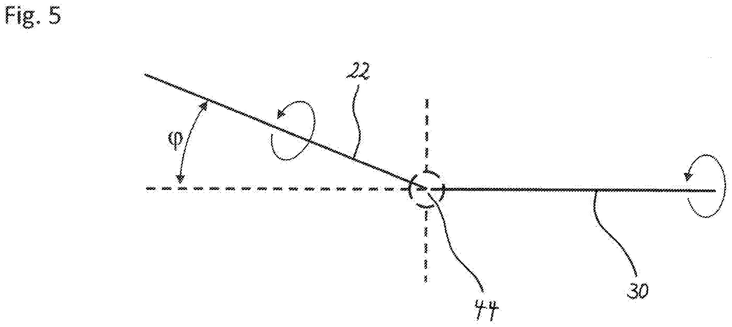

Bei den in den Fig. 1 bis 5 sowie 20 dargestellten Ausgestaltungen sind die Gehäusewellen 22 in von dem Instrumentenschaft 4 weg weisender Richtung schräggestellt. Insbesondere diese Ausrichtung der Gehäusewellen 22 und die damit einhergehende Ausrichtung der Betätigungsrollen 18 ermöglicht es, dass sich der Freiraum 14 in einer Ebene senkrecht zur Längsachse X des Instrumentenschaftes 4 in einem Winkel von 120° zu einer Spitze verjüngt. Korrespondierend hierzu spitzt sich auch das Gehäuseteil 16 in einem von dem Grundkörper 26 abgewandten Endabschnitt zu einem parallel zur Längsachse X des Instrumentenschaftes 4 verlaufenden First 38 zu. In unmittelbarer Nähe zu diesem First 38 mündet der Instrumentenschaft 4 in den Freiraum 14 des Instrumentengehäuses 12.

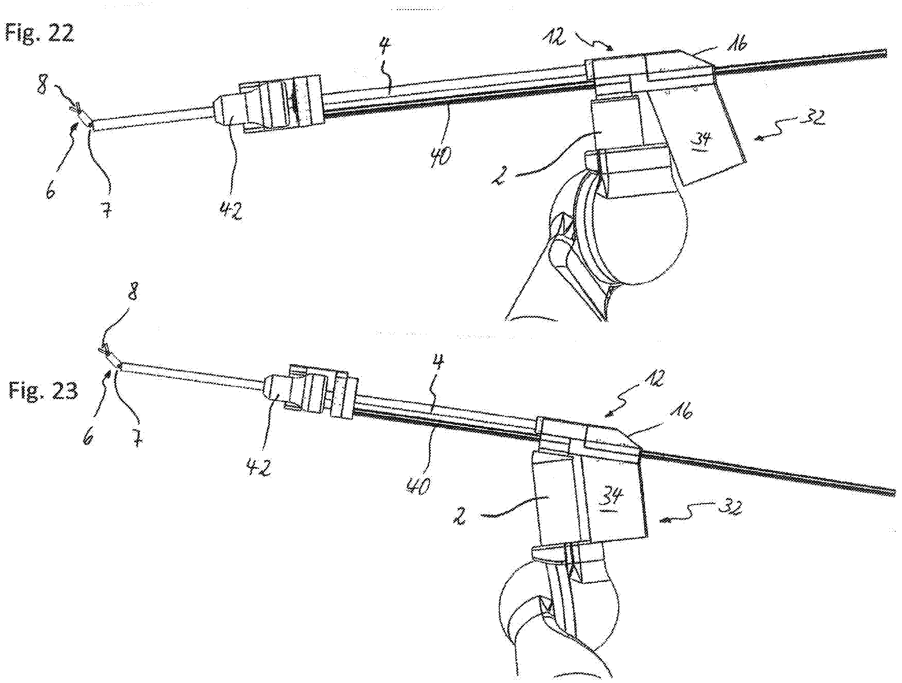

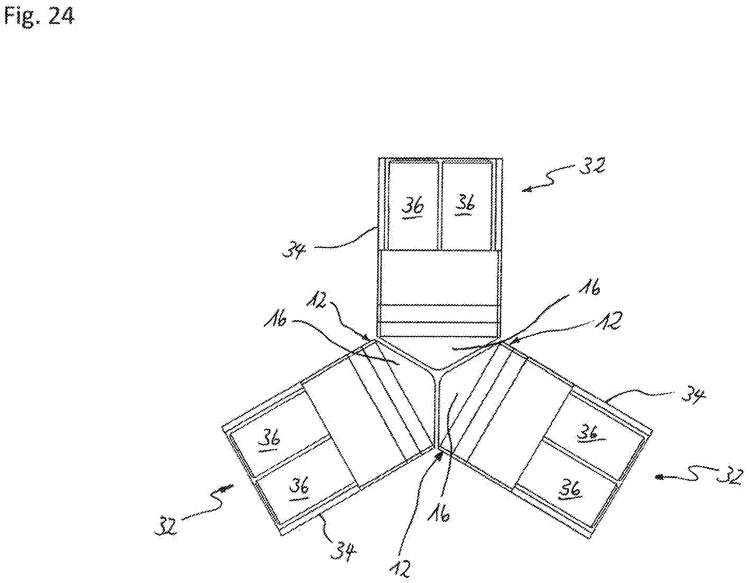

Die Vorteile dieser Ausgestaltung des Instrumentengehäuses 12 bzw. dessen Gehäuseteils 16 und der schrägen Ausrichtung der Gehäusewellen 22 werden aus den Fig. 20 und 24 deutlich. Wie in Fig. 24 dargestellt, ermöglicht es die Zuspitzung des Instrumentengehäuses 12 in einem Winkel von 120° zu einem First 38, drei Schaftinstrumente bei einem Single-Port-Eingriff gleichzeitig zu benutzen, da die Instrumentenschäfte 4 in unmittelbarer Nähe zueinander angeordnet werden können und somit gemeinsam über eine einzige Körperöffnung in den Körper eines Patienten eingeführt werden können. Aus Fig. 2 wird deutlich, dass durch die Anordnung und Ausrichtung der Gehäusewellen 22 mit den daran angeordneten Betätigungsrollen 18 zwischen den paarweise in Richtung der Längsachse X des Instrumentenschaftes 4 angeordneten Gehäusewellen 22 in dem Instrumentengehäuse 12 genügend Raum für eine parallel zur Längsachse X des Instrumentenschaftes 4 ausgerichtete Linearführung vorhanden ist, an deren Schiene 40 an dem distalen Ende ein Trokar 42 geführt sein kann, wobei der Instrumentenabschnitt 4 durch den Trokar 42 geführt ist (Fig. 22 und 23).

Neben einer Ausgestaltung, bei der die Gehäusewellen 22 in von dem Instrumentenschaft 4 weg weisender Richtung schräggestellt sind, besteht auch die Möglichkeit, die Gehäusewellen 22 in einer parallel zur Längsausdehnung des Instrumentenschaftes 4 ausgerichteten Ebene, in der die Antriebswellen 30 bzw. deren Längsachsen Y liegen, abgeschrägt auszurichten. Diese Ausrichtung der Gehäusewellen 22 schafft die Möglichkeit, den Instrumentenschaft 4 derart an dem Instrumentengehäuse 12 anzuordnen, dass, wie in den Fig. 21, 22 und 23 dargestellt, ein von dem Instrumentenschaft 4 und einer Mittelachse eines distalen Endabschnitts des Roboterarms 2 bzw. den Längsachsen Y der Antriebswellen 30 eingeschlossener Winkel von einem Winkel von 90° abweicht.

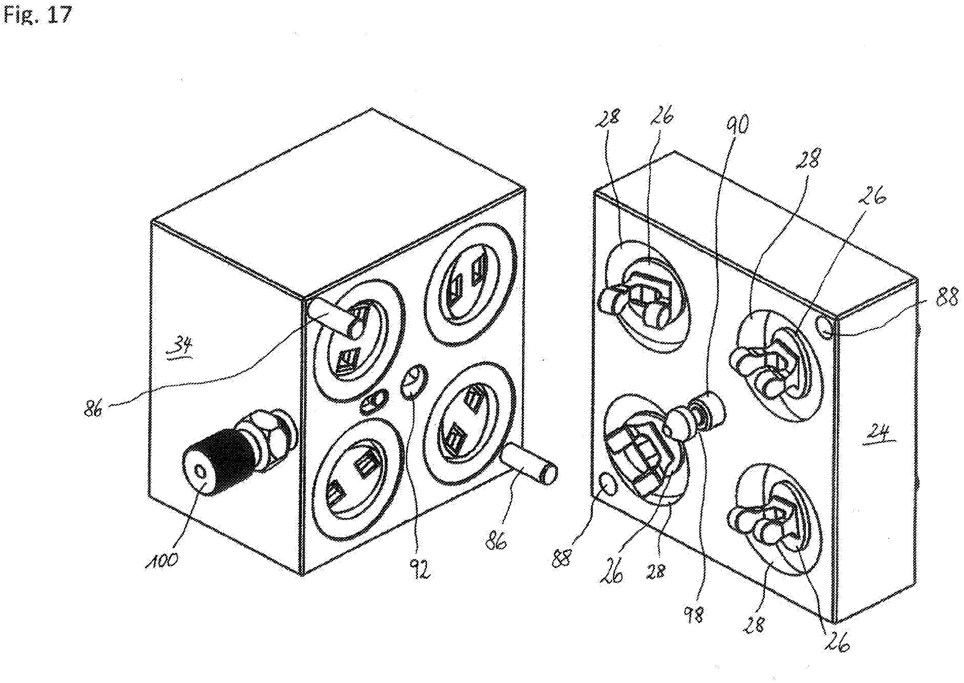

Die in Fig. 22 und Fig. 23 dargestellte Anordnung beschreibt die Befestigungsfolge von Roboterarm 2, Instrumentengehäuse 12 und Antriebseinheit 32. Das Instrumentengehäuse 12 ist wiederholt lösbar an einen ersten Bereich an den Roboterarm 2 angekoppelt und an einem zweiten Bereich an eine Antriebseinheit 32 angekoppelt. Die Antriebseinheit 32 stützt sich hierbei ausschließlich am Grundkörper 24 sowie dem Kardangelenk 44, 44' und dem Verschlußmechanismus (Fig. 17 bis Fig. 19) ab.

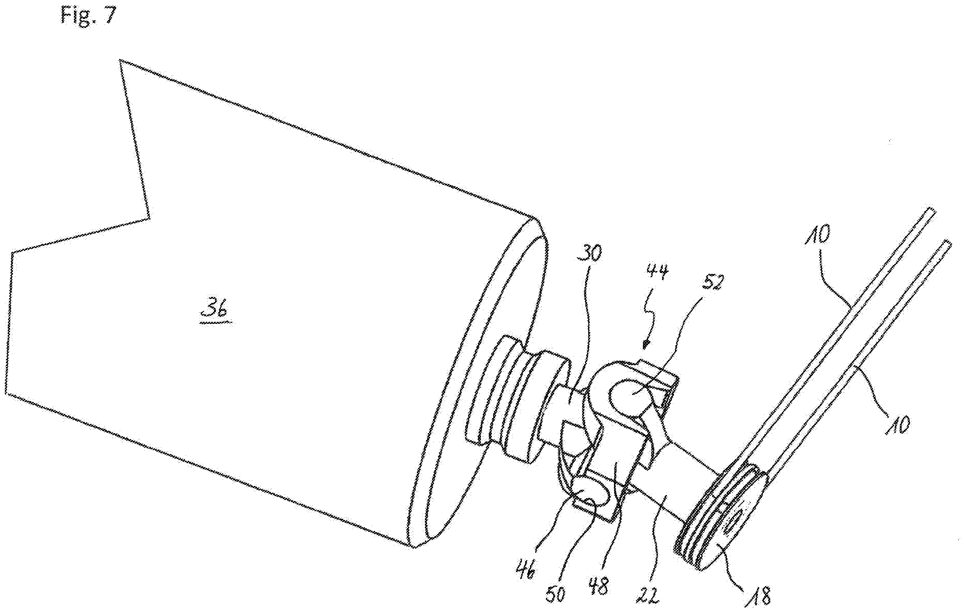

Die in dem Instrumentengehäuse 12 angeordneten Gehäusewellen 22 sind mit den Antriebswellen 30 der Antriebseinheit 32 über ein Kardangelenk 44 bzw. 44' antriebsverbindbar. Eine mögliche konstruktive Ausgestaltung eines Kardangelenks 44 ist in Fig. 7 dargestellt. Hier teilt sich das von dem Antriebsmotor 36 abgewandte Ende der Antriebswelle 30 gabelförmig in zwei Endabschnitte auf, wobei an den Enden der Endabschnitte Walzenkörper 46 ausgebildet sind, deren gemeinsame Mittelachse normal zur Längsachse Y der Antriebswelle 30 ausgerichtet ist. An der Antriebswelle 30 ist ein Taumelelement 48 angeordnet. Das Taumelelement 48 ist im Wesentlichen ringförmig ausgebildet, wobei an einer Stirnseite an dem Taumelelement 48 einander gegenüberliegend zwei mit der Form der Walzenkörper 46 korrespondierende Ausnehmungen 50 ausgebildet sind, in die die Walzenkörper 46 eingreifen. Durch einen an den beiden Ausnehmungen 50 ausgebildeten Hinterschnitt ist das Taumelelement 48 formschlüssig mit der Antriebswelle 30 verbunden, wobei es um eine von den Mittelachsen der Walzenkörper 46 gebildete Schwenkachse schwenkbar ist. Wie bereits angemerkt, teilen sich die zur Antriebsverbindung mit den Antriebswellen 30 vorgesehenen Enden der Gehäusewellen 22 ebenfalls gabelförmig in zwei Endabschnitte auf, wobei an den Enden der Endabschnitte Walzenkörper 52 ausgebildet sind, deren gemeinsame Mittelachse normal zur Längsachse Y der Gehäusewelle 22 ausgerichtet ist. Zur Aufnahme der an den Endabschnitt der Gehäusewelle 22 angeordneten Walzenkörper 52 sind an dem Taumelelement 48 an einer von den Ausnehmungen 50 abgewandten Stirnseite zu den Ausnehmungen 50 um 90° versetzt zwei Ausnehmungen 54 ausgebildet. In einem inneren Endbereich weisen diese Ausnehmungen 54 eine mit den Walzen körpern 52 korrespondierende Form auf, wobei sich die Ausnehmungen 54 aber in Richtung ihrer stirnseitigen Öffnung erweitern.

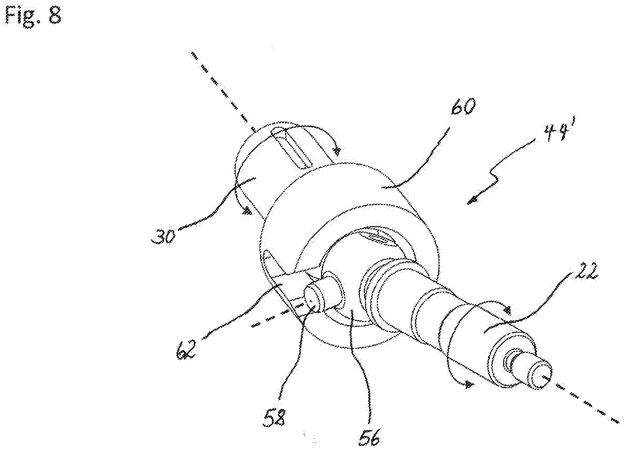

In Fig. 8 ist ein weiteres Ausführungsbeispiel eines bei der erfindungsgemäßen Antriebsanordnung verwendbaren Kardangelenks 44' dargestellt. Hier ist an dem an der von dem Freiraum 14 abgewandten Seite des Grundkörpers 24 herausragenden Ende der Gehäusewelle 22 ein Kugelkopf 56 ausgebildet, an dem ein zylindrischer Zapfen 58 quer zur Längsausdehnung der Gehäusewelle 22 herausragt. Am freien Ende der Antriebswelle 30 ist eine in Längsrichtung der Antriebswelle 30 offene Buchse 60 angeordnet. In diese Buchse 60 greift der an der Gehäusewelle 22 angeordnete Kugelkopf 56 ein, wobei der an dem Kugelkopf 56 angeordnete Zapfen 58 in einer an der Buchse 60 ausgebildeten Führungsnut 62 geführt ist, die sich ausgehend von dem offenen Ende der Buchse 60 in Längsrichtung der Antriebswelle 30 erstreckt.

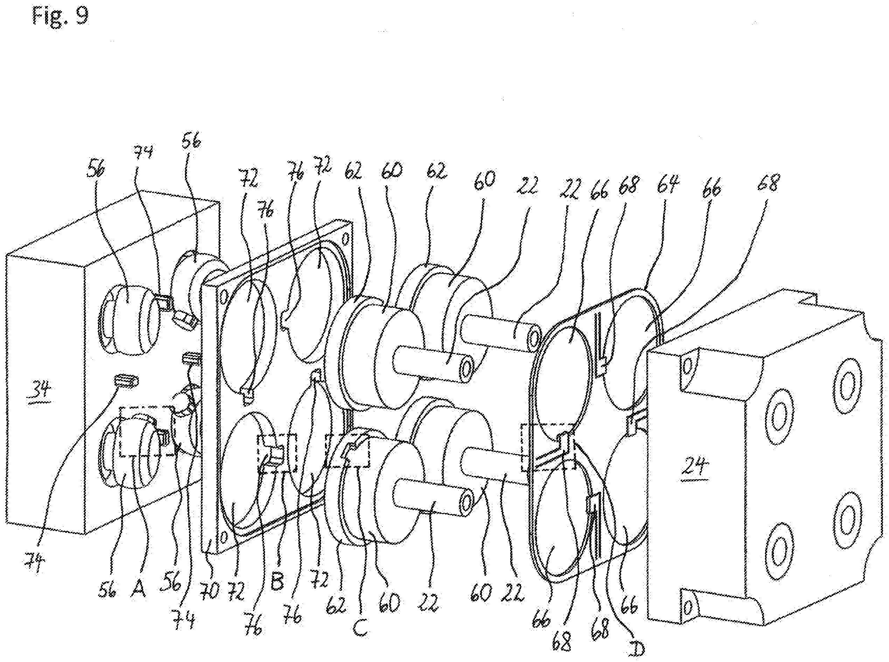

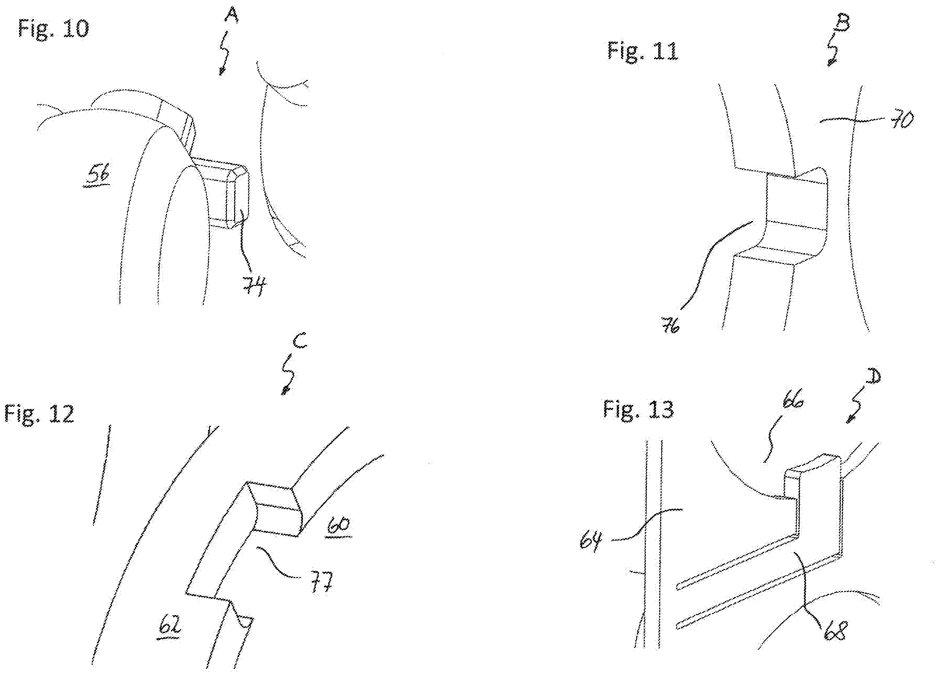

Den Fig. 9 bis 13 ist eine Ausgestaltung zu entnehmen, bei der im Instrumentengehäuse 12 gelagerten Gehäusewellen 22 bei von dem Antriebsgehäuse 34 getrenntem Instrumentengehäuse 12 mittels Federelementen in einer festgelegten Drehposition festlegbar sind. Hier sind an den von den Betätigungsrollen 18 abgewandten Enden der Gehäusewellen 22 Buchsen 60, die Teil eines Kardangelenks 40' bilden, angeordnet. An dem in Längsrichtung der Gehäusewellen 22 äußeren Ende der Buchsen 60 ist ein in radialer Richtung vorstehender ringförmiger Kragen 62 ausgebildet.

An der von dem Freiraum 14 abgewandten Außenseite des Grundkörpers 24 des Instrumentengehäuses 12 ist eine Federsegmentplatte 64 angeordnet. Die Federsegmentplatte 64 weist vier kreisförmige Ausnehmungen 66 auf, deren Lage und Größe mit den an dem Grundkörper 24 ausgebildeten Vertiefungen 28 korrespondiert. Ferner sind an der Federsegmentplatte vier L-förmige Blattfederelemente 68 ausgeschnitten, deren Endabschnitt jeweils in eine der Ausnehmungen 66 hineinragt. Die Federsegmentplatte 64 wird an dem Grundkörper 24 des Instrumentengehäuses 12 mittels einer Abdeckplatte 70 gehalten, die außenseitig der Federsegmentplatte 64 an dem Grundkörper 24 befestigt ist. Wie die Federsegmentplatte 64 weist auch die Abdeckplatte 70 vier kreisförmige Ausnehmungen 72 auf, deren Lage und Größe mit den an dem Grundkörper 24 ausgebildeten Vertiefungen 28 korrespondiert.

Die an den Gehäusewellen 22 ausgebildeten Buchsen 60 greifen in die an dem Grundkörper 24 ausgebildeten Vertiefungen 28 ein. Hierbei kommen die Blattfederelemente 68 an dem an den Buchsen 60 ausgebildeten Kragen 62 zur Anlage und werden hierdurch vorgespannt. Die Gehäusewellen 22 können manuell aber so verdreht werden, dass die Lage einer an dem Kragen 62 ausgebildeten Ausnehmung 72 mit der Lage des in die Ausnehmung 66 der Federsegmentplatte 64 hineinragenden Endes des Blattfederelements 68 übereinstimmt, so dass sich das Blattfederelement 68 entspannt und in die Ausnehmung 72 eingreift. Hierdurch werden die Gehäusewellen 22 gegen ein Verdrehen gesichert und in einer festgelegten Stellung gehalten.

An den aus dem Antriebsgehäuse 34 herausragenden Enden der Antriebswellen 30 sind Kugelköpfe 56 ausgebildet, die ebenfalls einen Teil eines Kardangelenks 44' bilden. Beim Zusammenfügen von Instrumentengehäuse 12 und Antriebsgehäuse 34 greifen die Kugelköpfe 56 in die an den Gehäusewellen 22 ausgebildeten Buchsen 60 ein. Gleichzeitig durchgreifen an dem Antriebsgehäuse 34 in axialer Richtung der Antriebswellen 30 vorstehende Stifte 74 Ausnehmungen 76, die an dem Außenrand der Ausnehmungen 72 der Abdeckplatte 70 ausgebildet sind und jeweils einen Bereich der Blattfederelemente 68 freilegen. Hierdurch werden die Blattfederelemente 68 aus den an dem Kragen 62 ausgebildeten Ausnehmung 77 herausgedrückt, so dass die Gehäusewellen 22 wieder frei drehbar sind.

Die Fig. 14 und 15 zeigen eine Ausgestaltung, bei der ein am freien Wellenende der Antriebswelle 30 von einer Hülse 78 gebildet wird, die sich über ein Federelement in Form einer Schraubenfeder 80 auf der Antriebswelle 30 abstützt. Um eine Verdrehung der Hülse 78 relativ zu der Antriebswelle 30 zu verhindern, ist an der Hülse 78 eine Längsnut 82 ausgebildet, in die ein an der Antriebswelle 30 angeordneter, radial ausgerichteter Stift 84 eingreift. Werden bei dieser Ausgestaltung das Instrumentengehäuse 12 und das Antriebsgehäuse 34 zusammengefügt, kontaktiert der Endabschnitt der Gehäusewelle 22, der einen Teil eines Kardangelenks 44 bildet, ein an der Hülse 78 angeordnetes Taumelelement 48, ohne dass der Endabschnitt der Gehäusewelle in an dem Taumelelement 48 ausgebildete Ausnehmungen eingreifen muss. Die Hülse 78 wird unter Vorspannung der Schraubenfeder 80 in axialer Richtung von dem Instrumentengehäuse weg verschoben. Durch Anfahren des mit der Antriebswelle 30 bewegungsgekoppelten Antriebsmotors 36 wird die Antriebswelle 30 so weit relativ gegenüber der Gehäusewelle 22 verdreht, bis sich der Endabschnitt der Antriebswelle 30 in einer Stellung befinden, in der der Endabschnitt der Gehäusewelle 22und das Taumelelement 48 eine Steckverbindung bilden können und die Hülse 78 mit dem Taumelelement 48 unter Entspannung der Schraubenfeder 80 in Richtung der Gehäusewelle 22 zurückbewegt wird.

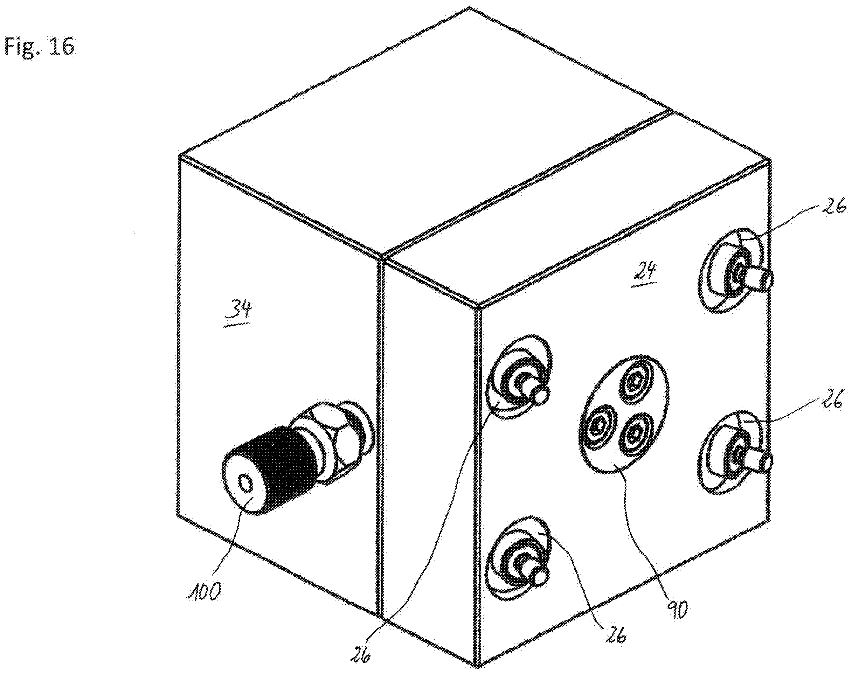

Aus den Fig. 16 bis 19 geht hervor, dass in einer speziellen Ausführungsform an der Fügefläche des Antriebsgehäuses 34 zwei Führungsstifte 86 angeordnet sind, die beim Zusammenfügen von Instrumentengehäuse 12 und Antriebsgehäuse 34 in an dem Instrumentengehäuse 12 ausgebildete Ausnehmungen 88 eingreifen. Ferner ist diesen Figuren zu entnehmen, dass an dem Grundkörper 24 des Instrumentengehäuses 12 ein Verschlussstift 90 befestigt ist, der durch eine an dem Grundkörper 24 ausgebildete, in Fügerichtung von Instrumentengehäuse 12 und Antriebsgehäuse 34 verlaufende Passbohrung geführt ist. Beim Zusammenfügen von Instrumentengehäuse 12 und Antriebsgehäuse 34 greift ein an der von dem Freiraum 14 abgewandten Seite des Grundkörpers 24 herausragender Endabschnitt des Verschlussstiftes 90 in eine an dem Antriebsgehäuse 34 ausgebildete Passbohrung 92 ein. An dem Antriebsgehäuse 34 ist eine weitere Passbohrung 94 ausgebildet, die die Passbohrung 92 kreuzt. In der Passbohrung 94 ist ein Verriegelungsstift 96 mit einem außerhalb des Antriebsgehäuses 34 angeordneten Betätigungskopf 100 axial verschiebbar geführt und in eine Stellung verschiebbar, in der er in eine an dem Verschlussstift 90 ausgebildete Ringnut 98 eingreift, wodurch die Verbindung von Antriebsgehäuse 34 und Instrumentengehäuse 12 gesichert wird.

### Bezugszeichenliste

- 2: - Roboterarm
- 4: - Instrumentenschaft
- 6: - Instrumentenkopf
- 7: - Abwinkelungskinematik
- 8: - Werkzeug
- 10: - Seilzug
- 12: - Instrumentengehäuse
- 14: - Freiraum
- 16: - Gehäuseteil
- 18: - Betätigungsrolle
- 20: - Umlenkrolle
- 22: - Gehäusewelle
- 24: - Grundkörper
- 26: - Lagerbuchse
- 28: - Vertiefung
- 30: - Antriebswelle
- 32: - Antriebseinheit
- 34: - Antriebsgehäuse
- 36: - Antriebsmotor
- 38: - First
- 40: - Schiene
- 42: - Trokar
- 44, 44': - Kardangelenk
- 46: - Walzenkörper
- 48: - Taumelelement
- 50: - Ausnehmung
- 52: - Walzenkörper
- 54: - Ausnehmung
- 56: - Kugelkopf
- 58: - Zapfen
- 60: - Buchse
- 62: - Kragen
- 64: - Federsegmentplatte
- 66: - Ausnehmung
- 68: - Blattfederelement
- 70: - Abdeckplatte
- 72: - Ausnehmung
- 74: - Stift
- 76: - Ausnehmung
- 77: - Ausnehmung
- 78: - Hülse
- 80: - Schraubenfeder
- 82: - Längsnut
- 84: - Stift
- 86: - Führungsstift
- 88: - Ausnehmung
- 90: - Verschlussstift
- 92: - Passbohrung
- 94: - Passbohrung
- 96: - Verriegelungsstift
- 98: - Ringnut
- 100: - Betätigungskopf

- A: - Einzelheit
- B: - Einzelheit
- C: - Einzelheit
- D: - Einzelheit
- X: - Längsachse
- Y: - Längsachse

## Patentansprüche

1. Antriebsanordnung für ein endoskopisches Schaftinstrument mit einem Instrumentengehäuse (12) am proximalen Schaftende, mit mindestens einer drehbar in dem Instrumentengehäuse (12) gelagerten Gehäusewelle (22), die mit Zugmitteln wiederholt lösbar antriebsverbunden ist, welche zur Steuerung eines Instrumentenkopfes (6) am distalen Schaftende vorgesehen ist, und mit einer Antriebseinheit (32) mit mindestens einer Antriebswelle (30), mit welcher die Gehäusewelle (22) antriebsverbindbar ist, **dadurch gekennzeichnet, dass** die zumindest eine Gehäusewelle (22) zu der mit ihr antriebsverbindbaren Antriebswelle (30) schräg ausgerichtet ist.

2. Antriebsanordnung nach Anspruch 1, **dadurch gekennzeichnet, dass** die Antriebseinheit (32) mit mindestens zwei in einem Winkel zueinander ausgerichteten Antriebswellen (30) mit gleicher Anzahl Gehäusewellen (22) antriebsverbindbar ist.

3. Antriebsanordnung nach Anspruch 1, **dadurch gekennzeichnet, dass** die Antriebseinheit (32) mit mindestens zwei parallel zueinander ausgerichteten Antriebswellen (30) mit gleicher Anzahl Gehäusewellen (22) antriebsverbindbar ist, wobei die Achsen der einzelnen Antriebswellen (30) und Gehäusewelle (22) innerhalb jeder paarweisen Zuordnung schräg ausgerichtet sind.

4. Antriebsanordnung nach einem der vorangehenden Ansprüche, **dadurch gekennzeichnet, dass** vier Gehäusewellen (22) paarweise in Richtung der Längsachse (X) des Instrumentenschaftes (4) hintereinander an zwei voneinander abgewandten Seiten des Instrumentenschaftes (4) angeordnet sind.

5. Antriebsanordnung nach einem der vorangehenden Ansprüche, **dadurch gekennzeichnet, dass** sich das Instrumentengehäuse (12) in einer Ebene normal zur Längsausdehnung des Instrumentenschaftes (4) zu einer Spitze verjüngt.

6. Antriebsanordnung nach einem der vorangehenden Ansprüche, **dadurch gekennzeichnet, dass** die Gehäusewelle(n) (22) in dem Instrumentengehäuse (12) in von dem Instrumentenschaft (4) wegweisender Richtung schräggestellt ist (sind).

7. Antriebsanordnung nach einem der vorangehenden Ansprüche, **dadurch gekennzeichnet, dass** die Gehäusewelle(n) (22) in dem Instrumentengehäuse (12) in einer parallel zur Längsausdehnung des Instrumentenschaftes (4) ausgerichteten Ebene, in der die Antriebswelle(n) (30) lieg(en)t, abgeschrägt ausgerichtet ist (sind).

8. Antriebsanordnung nach einem der vorangehenden Ansprüche, **dadurch gekennzeichnet, dass** die Gehäusewelle(n) (22) jeweils über zumindest ein Kardangelenk (44, 44') mit der(n) Antriebswelle(n) (30) bewegungsgekoppelt ist (sind).

9. Antriebsanordnung nach Anspruch 8, **dadurch gekennzeichnet, dass** das Kardangelenk (44, 44') einen mit der Gehäusewelle (22) verbundenen ersten Teil und einen mit der Antriebswelle (30) verbundenen zweiten Teil aufweist, wobei der erste und der zweite Teil des Kardangelenks (44, 44') mittels einer Steckverbindung miteinander verbindbar sind.

10. Antriebsanordnung nach einem der vorangehenden Ansprüche, **dadurch gekennzeichnet, dass** die Antriebseinheit (32) ein im Wesentlichen geschlossenes Antriebsgehäuse (34) aufweist, welches mit dem Instrumentengehäuse (12) mittels einer Steckverbindung verbindbar ist.

11. Antriebsanordnung nach Anspruch 10, **dadurch gekennzeichnet, dass** in dem Instrumentengehäuse (12) Federelemente vorgesehen sind, mit welchen die darin gelagerten Gehäusewellen (22) bei von dem Antriebsgehäuse (34) getrenntem Instrumentengehäuse (12) in einer festgelegten Drehposition festlegbar sind.

12. Antriebsanordnung nach Anspruch 11, **dadurch gekennzeichnet, dass** in dem Instrumentengehäuse (12) jeder der dort angeordneten Gehäusewellen (22) ein Blattfederelement (68) zugeordnet ist, welches jeweils in einer Verriegelungsstellung in eine im Bereich der Außenseite des an der jeweiligen Gehäusewelle (18) angeordneten ersten Teil des Kardangelenks (44, 44') ausgebildete Ausnehmung (72) eingreift.

13. Antriebsanordnung nach Anspruch 12, **dadurch gekennzeichnet, dass** an dem Antriebsgehäuse (34) in Fügerichtung von Instrumentengehäuse (12) und Antriebsgehäuse (34) vorstehende Vorsprünge ausgebildet sind, welche beim Zusammenfügen von Instrumentengehäuse (12) und Antriebsgehäuse (34) die in dem Instrumentengehäuse (12) vorgesehenen Blattfederelemente (68) in eine Entriegelungsstellung bewegen.

14. Antriebsanordnung nach Anspruch 10, **dadurch gekennzeichnet, dass** die Gehäusewellen (22) in dem Instrumentengehäuse (12) in axialer Richtung auf Federelementen gelagert sind und gegen Federkraft entgegen der Fügerichtung von Instrumentengehäuse (12) und Antriebsgehäuse (34) axial verschiebbar sind.

15. Antriebsanordnung nach Anspruch 10, **dadurch gekennzeichnet, dass** die Antriebswellen (30) in dem Instrumentengehäuse (12) in axialer Richtung auf Federelementen gelagert sind und gegen Federkraft entgegen der Fügerichtung von Instrumentengehäuse (12) und Antriebsgehäuse (34) axial verschiebbar sind.

16. Antriebsanordnung nach einem der Ansprüche 10 bis 15, **dadurch gekennzeichnet, dass** an den Fügeflächen von Instrumentengehäuse (12) und Antriebsgehäuse (34) Führungsmittel zur Lagefixierung des Antriebsgehäuses (34) an dem Instrumentengehäuse (12) vorgesehen sind.

17. Antriebsanordnung nach einem der Ansprüche 10 bis 16, **dadurch gekennzeichnet, dass** an dem Antriebsgehäuse (34) eine in Fügerichtung von Instrumentengehäuse (12) und Antriebsgehäuse (34) durch das Instrumentengehäuse (12) verlaufende Passbohrung ausgebildet ist und an dem Antriebsgehäuse (34) ausgehend von einer Fügefläche mit dem Instrumentengehäuse (12) eine korrespondierende Passbohrung (92) ausgebildet ist, wobei die an dem Antriebsgehäuse (34) und dem Instrumentengehäuse (12) ausgebildeten Passbohrungen zur Aufnahme eines Verschlussstiftes (90) vorgesehen sind.

18. Antriebsanordnung nach Anspruch 17, **dadurch gekennzeichnet, dass** an dem Instrumentengehäuse (12) eine die dort in Fügerichtung von Instrumentengehäuse (12) und Antriebsgehäuse (34) verlaufende Passbohrung (92) kreuzende zweite Passbohrung (94) ausgebildet ist, in welcher ein Verriegelungsstift (96) verschiebbar geführt ist, der in einer Verriegelungsstellung in eine an dem Verschlussstift (90) ausgebildete Ausnehmung eingreift.

## Claims

1. A drive arrangement for an endoscopic shank instrument with an instrument housing (12) at the proximal shank end, with at least one housing shaft (22) which is rotatably mounted in the instrument housing (12) and which is repeatedly releasably drive-connected to pull means, said pull means being provided at the distal shank end for the control of an instrument head (6), and with a drive unit (32) with at least one drive shaft (30), to which drive shaft the housing shaft (22) is drive-connectable, **characterised in that** the at least one housing shaft (22) is aligned obliquely to the drive shaft (30) which is drive-connectable to it.

2. A drive arrangement according to claim 1, **characterised in that** the drive unit (32) with at least two drive shafts (30) which are aligned at an angle to one another is drive-connectable to an equal number of housing shafts (22).

3. A drive arrangement according to claim 1, **characterised in that** the drive unit (32) with at least two drive shafts (30) which are aligned parallel to one another is drive-connectable to an equal number of housing shafts (22), wherein the axes of the individual drive shaft (30) and housing shaft (22) within each paired assignment are aligned in an oblique manner.

4. A drive arrangement according to one of the preceding claims, **characterised in that** four housing shafts (22) are arranged in pairs successively in the direction of the longitudinal axis (X) of the instrument shank (4), at two sides of the instrument shank (4) which are away from one another.

5. A drive arrangement according to one of the preceding claims, **characterised in that** the instrument housing (12) tapers in a plane normal to the longitudinal extension of the instrument shank (4), into a tip.

6. A drive arrangement according to one of the preceding claims, **characterised in that** the housing shaft(s) (22) in the instrument housing is (are) set obliquely in a direction which points away from the instrument shank (4).

7. A drive arrangement according to one of the preceding claims, **characterised in that** the housing shaft(s) (22) in the instrument housing (12) is(are) aligned in a slanted manner in a plane which is aligned parallel to the longitudinal extension of the instrument shank (4) and in which the drive shaft(s) (30) lie.

8. A drive arrangement according to one of the preceding claims, **characterised in that** the housing shaft(s) (22) is (are) coupled in movement to the drive shaft(s) (30) in each case via at least one universal joint (44, 44').

9. A drive arrangement according to claim 8, **characterised in that** the universal joint (44, 44') comprises a first part which is connected to the housing shaft (22) and a second part which is connected to the drive shaft (30), wherein the first and the second part of the universal joint (44, 44') are connectable to one another by way of a plug-in connection.

10. A drive arrangement according to one of the preceding claims, **characterised in that** the drive unit (32) has an essentially closed drive housing (34) which is connectable to the instrument housing (12) by way of a plug-in connection.

11. A drive arrangement according to claim 10, **characterised in that** spring elements are provided in the instrument housing (12), with which spring elements the housing shafts (22) which are mounted therein can be fixed in a fixed rotational position given an instrument housing (12) separated from the drive housing (34).

12. A drive arrangement according to claim 11, **characterised in that** in the instrument housing (12), a leaf spring element (68) is assigned to each of the housing shafts (22) which are arranged there, said leaf spring element in a locking position in each case engaging into recess (72) which is formed in the region of the outer side of the first part of the universal joint (44, 44'), said first part being arranged on the respective housing shaft (18).

13. A drive arrangement according to claim 12, **characterised in that** projections which project in the joining direction of the instrument housing (12) and the drive housing (34) are formed on the drive housing (34) and on joining together the instrument housing (12) and the drive housing (34) move the leaf spring elements (68) which are provided in the instrument housing (12) into an unlocking position.

14. A drive arrangement according to claim 10, **characterised in that** the housing shafts (22) in the instrument housing (12) are mounted in the axial direction on spring elements and are axially displaceable against spring force counter to the joining direction of the instrument housing (12) and the drive housing (34).

15. A drive arrangement according to claim 10, **characterised in that** the drive shafts (30) in the drive housing (12) are mounted in the axial direction on springs elements and are axially displaceable against spring force counter to the joining direction of the instrument housing (12) and drive housing (34).

16. A drive arrangement according to one of the claims 10 to 15, **characterised in that** guide means for the positional fixation of the drive housing (34) on the instrument housing (12) are provided on the joining surfaces of the instrument housing (12) and drive housing (34).

17. A drive arrangement according to one of the claims 10 to 16, **characterised in that** a fitting bore which runs through the instrument housing (12) in the joining direction of the instrument housing (12) and the drive housing (34) is formed on the drive housing (34), and a corresponding fitting bore (92) is formed on the drive housing (34) in a manner departing from a surface joining to the instrument housing (12), wherein the fitting bores which are formed on the drive housing (34) and the instrument housing (12) are provided for receiving a closure pin (90).

18. A drive arrangement according to claim 17, **characterised in that** a second fitting bore (94) is formed on the instrument housing (12) and crosses the fitting bore (92) which runs there in the joining direction of the instrument housing (10) and drive housing (30), in which second fitting bore a locking pin (96) is displaceably guided, said locking pin in a locking position engaging into a recess formed on the closure pin (90).

## Revendications

1. Dispositif d'entraînement pour un instrument endoscopique à tige avec un boîtier d'instrument (12) à l'extrémité proximale de la tige, avec au moins un arbre de boîtier (22) monté tournant dans le boîtier d'instrument (12), lequel est solidaire en entraînement, de façon détachable à répétition, de moyens de traction qui sont prévus pour la commande d'une tête d'instrument (6) à l'extrémité distale de la tige, et avec une unité d'entraînement (32) avec au moins un arbre d'entraînement (30) avec lequel l'arbre de boîtier (22) peut être rendu solidaire en entraînement, **caractérisé en ce que** ledit au moins un arbre de boîtier (22) est orienté de façon oblique par rapport à l'arbre d'entraînement (30) susceptible d'être rendu solidaire en entraînement avec celui-ci.

2. Dispositif d'entraînement selon la revendication 1, **caractérisé en ce que** l'unité d'entraînement (32) est adaptée pour être rendue solidaire en entraînement avec au moins deux arbres d'entraînement (30) orientés les uns par rapport aux autres sous un angle et ayant le même nombre d'arbres de boîtier (22).

3. Dispositif d'entraînement selon la revendication 1, **caractérisé en ce que** l'unité d'entraînement (32) est adaptée pour être rendue solidaire en entraînement avec au moins deux arbres d'entraînement (30) orientés parallèlement les uns aux autres et ayant le même nombre d'arbres de boîtier (22), les axes de chacun des arbres d'entraînement (30) et d'arbres de boîtier (22) étant orientés de façon oblique l'un par rapport à l'autre à l'intérieur de chaque association par paire.

4. Dispositif d'entraînement selon l'une des revendications précédentes, **caractérisé en ce que** quatre arbres de boîtier (22) sont disposés par paires dans la direction de l'axe longitudinal (X) de la tige d'instrument (4) les uns derrière les autres à deux côtés opposés de la tige d'instrument (4).

5. Dispositif d'entraînement selon l'une des revendications précédentes, **caractérisé en ce que** le boîtier d'instrument (12) se rétrécit dans un plan perpendiculaire à l'étendue longitudinale de la tige d'instrument (4) vers une pointe.

6. Dispositif d'entraînement selon l'une des revendications précédentes, **caractérisé en ce que** le (ou les) arbre(s) de boîtier (22) dans le boîtier d'instrument (12) est (sont) disposé(s) en oblique dans une direction éloignant de la tige d'instrument (4).

7. Dispositif d'entraînement selon l'une des revendications précédentes, **caractérisé en ce que** le (ou les) arbre(s) de boîtier (22) dans le boîtier d'instrument (12) est (sont) orienté(s) en oblique dans un plan parallèle à l'étendue de la tige d'instrument (4) dans lequel sont situés les arbres d'entraînement (30).

8. Dispositif d'entraînement selon l'une des revendications précédentes, **caractérisé en ce que** le (ou les) arbre(s) de boîtier (22) est (sont) respectivement solidaire(s) en mouvement avec le (les) arbre(s) d'entraînement (30) par au moins un joint universel (44, 44').

9. Dispositif d'entraînement selon la revendication 8, **caractérisé en ce que** le joint universel (44, 44') comprend une première partie attachée à l'arbre de boîtier (22) et une seconde partie attachée à l'arbre d'entraînement (30), la première et la seconde partie du joint universel (44, 44') étant adaptées pour être attachées l'une à l'autre à l'aide d'une connexion enfichable.

10. Dispositif d'entraînement selon l'une des revendications précédentes, **caractérisé en ce que** l'unité d'entraînement (32) comprend un boîtier d'entraînement (34) essentiellement fermé qui est susceptible d'être relié au boîtier d'instrument (12) à l'aide d'une connexion enfichable.

11. Dispositif d'entraînement selon la revendication 10, **caractérisé en ce que** sont prévus, dans le boîtier d'instrument (12), des éléments ressort à l'aide desquels les arbres de boîtier (22) qui y sont montés, peuvent être maintenus dans une position déterminée de rotation en cas de boîtier d'instrument (12) séparé du boîtier d'entraînement (34).

12. Dispositif d'entraînement selon la revendication 11, **caractérisé en ce qu'**il est associé, dans le boîtier d'instrument (12), à chacun des arbres de boîtier (22) qui y sont disposés, un élément de ressort à lame (68) qui s'engage dans une position de verrouillage respectivement dans un évidement (72) formé dans la zone de la face extérieure de la première partie du joint universel (44, 44') disposée à l'arbre de boîtier (18) respectif.

13. Dispositif d'entraînement selon la revendication 12, **caractérisé en ce que** des saillies sont formées sur le boîtier d'entraînement (34) dans la direction d'assemblage du boîtier d'instrument (12) et du boîtier d'entraînement (34), qui déplacent les éléments ressort à lame (68) prévus dans le boîtier d'instrument (12), lors de l'assemblage du boîtier d'instrument (12) et du boîtier d'entraînement (34), dans une position de déverrouillage.

14. Dispositif d'entraînement selon la revendication 10, **caractérisé en ce que** les arbres de boîtier (22) sont montés dans le boîtier d'instrument (12) dans la direction axiale sur des éléments ressort et déplaçables axialement dans le sens contraire à la direction d'assemblage du boîtier d'instrument (12) et du boîtier d'entraînement (34) et à l'encontre d'un effort élastique.

15. Dispositif d'entraînement selon la revendication 10, **caractérisé en ce que** les arbres d'entraînement (30) sont montés dans le boîtier d'instrument (12) dans la direction axiale sur des éléments ressort et déplaçables axialement dans le sens contraire à la direction d'assemblage du boîtier d'instrument (12) et du boîtier d'entraînement (34) et à l'encontre d'un effort élastique.

16. Dispositif d'entraînement selon l'une des revendications 10 à 15, **caractérisé en ce que** des moyens de guidage sont prévus sur les surfaces d'assemblage du boîtier d'instrument (12) et du boîtier d'entraînement (34), pour définir la position du boîtier d'entraînement (34) sur le boîtier d'instrument (12).

17. Dispositif d'entraînement selon l'une des revendications 10 à 16, **caractérisé en ce qu'**un alésage de positionnement est formé sur le boîtier d'entraînement (34) dans la direction d'assemblage du boîtier d'instrument (12) et du boîtier d'entraînement (34) et passant par le boîtier d'instrument (12) et qu'un alésage de positionnement (92) correspondant est formé sur le boîtier d'entraînement (34) partant de la surface d'assemblage avec le boîtier d'instrument (12), les alésages des positionnement formés sur le boîtier d'entraînement (34) et le boîtier d'instrument (12) étant prévus pour la réception d'un pointeau de fermeture (90).

18. Dispositif d'entraînement selon la revendication 17, **caractérisé en ce qu'**un deuxième alésage de positionnement (94) est formé au boîtier d'instrument (12), croisant l'alésage de positionnement (92) qui s'y étend dans la direction d'assemblage du boîtier d'instrument (12) et du boîtier d'entraînement (34), dans lequel un pointeau de verrouillage (96) est guidé de façon déplaçable qui s'engage, en une position de verrouillage, dans un évidement formé sur le pointeau de fermeture (90).
